(19) **Europäisches Patentamt**

**European Patent Office**

**Office européen des brevets**

(11) **EP 1 645 230 A2**

(12) **EUROPÄISCHE PATENTANMELDUNG**

(43) Veröffentlichungstag:
**12.04.2006 Patentblatt 2006/15**

(51) Int Cl.:
***A61B 5/11*** *(2006.01)*

(21) Anmeldenummer: **05019892.8**

(22) Anmeldetag: **13.09.2005**

(84) Benannte Vertragsstaaten:
**AT BE BG CH CY CZ DE DK EE ES FI FR GB GR HU IE IS IT LI LT LU LV MC NL PL PT RO SE SI SK TR**
Benannte Erstreckungsstaaten:
**AL BA HR MK YU**

(30) Priorität: **17.09.2004 DE 102004045141**

(71) Anmelder: **Wacker Construction Equipment AG**
**80809 München (DE)**

(72) Erfinder: **Stenzel, Otto W. , Dr.**
**82211 Herrsching. (DE)**

(74) Vertreter: **Müller - Hoffmann & Partner**
**Patentanwälte,**
**Innere Wiener Strasse 17**
**81667 München (DE)**

(54) **Expositionsmessvorrichtung für ein Arbeitsgerät**

(57)    Eine Expositionsmessvorrichtung für ein Arbeitsgerät (1) weist wenigstens einen Beschleunigungsaufnehmer (5) auf, der an einer Körperkontaktstelle (4) angeordnet ist, an der ein Bediener das Arbeitsgerät (1) durch Halten, Führen oder Aufsitzen berührt. Die von dem Beschleunigungsaufnehmer (5) erfassten Beschleunigungswerte werden an eine Auswerteeinrichtung (6) übermittelt, die durch Integrieren eine aktuelle Gesamt-Expositionsinformation bestimmt. Die Gesamt-Expositionswerte können gespeichert oder dem Bediener angezeigt werden. Auf diese Weise erhält der Bediener oder sein Arbeitgeber Informationen darüber, welchen Schwingungen der Bediener beim Betrieb des Arbeitsbetriebs exponiert war.

**Beschreibung**

Die Erfindung betrifft eine Expositionsmessvorrichtung für ein Arbeitsgerät.

Es sind handgehaltene bzw. handgeführte Arbeitsgeräte bekannt, wie z. B. Bohr- und/oder Schlaghämmer, motorbetriebene Sägen, Fugenschneider, Vibrationsplatten und Stampfer zur Bodenverdichtung sowie Winkelschleifer, Rasentrimmer, Heckenscheren, Laubsauger usw. Andere Arbeitsgeräte können als Aufsitzer betrieben werden, wie z. B. Walzen zur Bodenverdichtung, Beton-Flügelglätter, Lader, Grader sowie alle Arten von Kraftfahrzeugen und Nutzkraftwagen. Die beim Betrieb der Arbeitsgeräte entstehenden Schwingungen können für den Bediener je nach Stärke und Dauer der Einwirkung (Exposition) gesundheitsschädliche Auswirkungen haben.

Zum Schutz des Bedieners wurden eine ganze Reihe von Vorschriften verfasst, die Grenzen für Hand-Arm-Schwingungen oder Ganzkörper-Vibrationen vorgeben. So werden z. B. mit der Richtlinie 2002/44/EG des Europäischen Parlaments und des Rates vom 25. Juni 2002 Mindestanforderungen für den Schutz der Arbeitnehmer gegen tatsächliche oder mögliche Gefährdungen ihrer Gesundheit und Sicherheit durch Einwirkung von Vibrationen während ihrer Arbeit festgelegt. Die Normen DIN EN ISO 5349-1 und -2 enthalten Vorschriften für die Messung und Bewertung der Einwirkung von Schwingungen auf das Hand-Arm-System des Menschen.

Aufgrund der zahlreichen Vorschriften zeichnet es sich ab, dass der Einsatz von schwingungserzeugenden Arbeitsgeräten und die dadurch resultierende Belastung des Bedieners in zunehmendem Maße insbesondere durch den Arbeitgeber überwacht werden muss. Aufgrund der Komplexität der Messverfahren (siehe z. B. DIN EN ISO 5349-1 und -2) ist der Aufwand zur Erfassung der tatsächlichen Exposition des Bedieners hoch. Arbeitgeber könnten verpflichtet werden, auf Baustellen entsprechende Überwachungen einzurichten, was mit einem großen Bedarf an qualifiziertem Personal einhergehen wird.

Es ist bekannt, Arbeitsgeräte mit Beschleunigungsaufnehmern, insbesondere an den Handgriffen, auszustatten und während eines Testbetriebs die wirksamen Beschleunigungen zu messen. Daraus können Mittelwerte gebildet werden, die als Grundlage für eine Abschätzung der Exposition des Bedieners gegenüber Vibrationen genommen werden.

Das einfache Hochrechnen einer derart gemessenen und gemittelten Vibration eines Arbeitsgeräts unter Berücksichtigung z. B. der an einem Arbeitstag geleisteten Gesamtdauer des Einsatzes eines Arbeitsgeräts ist zu unpräzise, als dass es die realen Einsatzbedingungen nachbilden könnte. Zum Beispiel sind die Schwingungen an einem Führungsgriff eines Arbeitsgeräts ungleichmäßig. Exemplarisch ermittelte Schwingungswerte gelten oft nur für eine intensive Nutzung, die in der Praxis so meist nicht erfolgt. Bei entsprechender Hochrechnung würden somit Expositionswerte berechnet, die weit über den tatsächlichen Expositionswerten liegen würden. Dementsprechend würden Arbeitskräfte weniger intensiv eingesetzt, als es die Gesetzeslage und medizinische Erkenntnisse zulassen würden, wodurch letztendlich zusätzliche Personalkosten entstehen würden.

Gerade bei Arbeitsprozessen mit Hämmern oder Stampfern treten immer wieder Arbeitspausen auf, in denen die Geräte neu positioniert oder - bei Stampfern - Erde nachgefüllt werden muss. Bei Vibrationsplatten, Walzen oder Fugenschneidern können die Deichselbewegungen bei verdichteten harten Böden stärker sein als bei unverdichteten, weichen Böden.

Der Erfindung liegt die Aufgabe zugrunde, eine Expositionsmessvorrichtung für ein Arbeitsgerät anzugeben, mit der es möglich ist, in möglichst einfacher Weise die tatsächlich auf den Bediener einwirkenden Schwingungen zu erfassen und auszuwerten.

Die Aufgabe wird erfindungsgemäß durch eine Expositionsmessvorrichtung gemäß Patentanspruch 1 gelöst. Vorteilhafte Weiterentwicklungen sind in den abhängigen Ansprüchen definiert.

Demnach weist eine erfindungsgemäße Expositionsmessvorrichtung für ein Arbeitsgerät wenigstens einen Beschleunigungsaufnehmer auf, der an einer Körperkontaktstelle angeordnet ist, an der ein Bediener das Arbeitsgerät durch Halten, Führen oder Aufsitzen berührt, wobei der Beschleunigungsaufnehmer zum Erfassen eines aktuellen Beschleunigungswertes an der Körperkontaktstelle dient. Auf diese Weise können die auf den Bediener einwirkenden Schwingungen gemessen werden. Weiterhin ist eine Auswerteeinrichtung vorgesehen, zum fortlaufenden Bestimmen einer Expositionsinformation aufgrund der von dem Beschleunigungsaufnehmer erfassten Beschleunigungswerte über einer Zeitdauer der Beschleunigung und zum Summieren oder Integrieren der Expositionsinformationen zu einer aktuellen Gesamt-Expositionsinformation. Die gewonnenen Werte zu der aktuellen Gesamt-Expositionsinformation und/oder einzelner Expositionsinformationen sind in einer Speichereinrichtung speicherbar. Die Expositionsmessvorrichtung ist in das Arbeitsgerät voll integriert, so dass die Schwingungen im Betrieb ständig erfasst und ausgewertet werden können. Die bisher übliche Messung von Schwingungen eines Arbeitsgeräts während eines gesonderten Messzyklus - meist im Testbetrieb - kann somit entfallen. Vielmehr werden die tatsächlich auf den Bediener einwirkenden Schwingungen permanent erfasst und ausgewertet.

Die Auswertung, insbesondere die Integration der Expositionsinformationen kann in an sich bekannter Weise erfolgen und ist z. B. in den Normen DIN EN ISO 5349-1 und -2 beschrieben. Die tatsächliche "Expositionsleistung" (physikalisch: eine der Expositionsenergie proportionale Größe) an der Körperkontaktstelle, also z. B. an einem Handgriff, wird bestimmt, indem die tatsächlich am Handgriff auftretenden Beschleunigungsleistungen über der Zeit integriert werden.

Die Beschleunigungssignale der Komponenten können jeweils getrennt mit einem Bewertungsfilter umgerechnet werden und dann anschließend vektoriell addiert werden. Es ist aber auch möglich, zunächst die Signale vektoriell zu addieren und dann zu bewerten, bevor sie quadriert über der Zeit aufintegriert werden.

In den Normen sind entsprechende Filter festgelegt. Die Filterung kann bereits im Analogen wie auch im Digitalen erfolgen, wobei neben der Amplitudenbewertung auch die Phase berücksichtigt werden muss. Erst anschließend werden die Signale quadriert und integriert.

Die Effektivwertbildung mit Quadrierung und anschließender Integration führen zum gleichen Ergebnis.

Durch das Speichern der Gesamt-Expositionsinformation, also insbesondere der gesamten "Expositionsleistung" z. B. während eines Arbeitstages, kann die Exposition des Bedieners stets ermittelt und abgefragt werden. Auf diese Weise kann der Arbeitgeber des Bedieners ständig überwachen, dass Arbeitsschutzgesetze eingehalten werden, und gegebenenfalls Gegenmaßnahmen ergreifen, zu denen ihn z. B. die oben genannte Richtlinie 2002/44/EG verpflichtet.

Vorteilhafterweise sind an der Körperkontaktstelle mehrere Beschleunigungsaufnehmer angeordnet, wobei die damit erfassten Beschleunigungswerte durch die Auswerteeinrichtung zu einer einzigen Expositionsinformation zusammengefasst werden können. Durch die mehreren Beschleunigungsaufnehmer ist es möglich, Beschleunigungen in mehreren Raumrichtungen zu erfassen. Eine resultierende Beschleunigung lässt sich dann z. B. durch die Wurzel aus der Summe der Quadrate der Beschleunigungen in die einzelnen Raumrichtungen erfassen.

Bei einer besonders vorteilhaften Ausführungsform der Erfindung ist eine Anzeigeeinrichtung vorgesehen, zum Anzeigen eines Zustands der aktuellen Gesamt-Expositionsinformation. Auf diese Weise lässt sich bereits am Arbeitsgerät direkt ablesen, welcher Gesamt-Exposition der Bediener aktuell ausgesetzt ist.

Als Zustand für die aktuelle Gesamt-Expositionsinformation können verschiedene Anzeige-Optionen gewählt werden. So genügt z. B. unter Umständen das einfache Anzeigen eines Unter- oder Überschreitens eines Grenzwertes für eine zulässige Gesamt-Exposition, also eines maximal zulässigen Gesamt-Expositionswerts, durch die aktuelle Gesamt-Expositionsinformation. Dieser Wert wird z. B. in der Richtlinie 2002/44/EG als "täglicher Expositionsgrenzwert" bezeichnet. Solange der Grenzwert noch nicht erreicht ist, wird dem Bediener "grünes Licht" gezeigt bzw. keine Information mitgeteilt. Erst bei Überschreiten des Grenzwertes oder eines vorgelagerten Warnwertes erhält der Bediener die Information. Dies kann optisch (z. B. durch eine rote Warnleuchte) erfolgen, aber auch akustisch durch einen Warnton. Je nach gewünschter Ausgestaltung des Arbeitsgeräts kann der Warnton derart penetrant werden, dass ein Weiterarbeiten für den Bediener deutlich erschwert wird.

Ergänzend oder alternativ dazu kann durch die Anzeigeeinrichtung ein der aktuellen Gesamt-Expositionsinformation entsprechender Zahlenwert ausgegeben werden. Die genannte EU-Richtlinie sowie die oben zitierten Normen geben Zahlen-Grenzwerte vor, gegenüber denen die auf dem Arbeitsgerät angezeigten Zahlenwerte abgeglichen werden können. Zum Beispiel wird für die Hand-Arm-Belastung nach der EU-Richtlinie der tägliche Expositionsgrenzwert, normiert auf einen Bezugszeitraum von 8 Stunden, auf $5\,m/s^2$ festgesetzt. Damit ergibt sich ein maximal zulässiger Grenzwert für die tägliche Exposition von $200\,m^2/s^4$ x h ($=5^2\,m^2/s^4$ x 8 h je Tag).

Die Gesamt-Exposition ergibt sich z. B. aus der Summe der mit den jeweiligen Zeitabschnitten multiplizierten quadrierten Beschleunigungswerte.

Ebenfalls alternativ dazu kann auch ein Verhältnis des Zahlenwerts der aktuellen Gesamt-Expositionsinformation zu dem maximal zulässigen Gesamt-Expositionswert angezeigt werden, wobei sich daraus auch ohne weiteres eine entsprechende Restbetriebszeit des Arbeitsgeräts bis zum Erreichen des maximal zulässigen Gesamt-Expositionswerts unter der Annahme eines im Wesentlichen gleichbleibenden Schwingungsschemas ermitteln lässt. Die Restbetriebszeit wird dann ähnlich einer Reichweitenanzeige bei einem Kfz bestimmt, bei dem auf Grund des bisherigen und des aktuellen Kraftstoffverbrauchs sowie des noch im Tank verfügbaren Kraftstoffs die noch erreichbare Reichweite ständig neu ermittelt wird.

Besonders vorteilhaft ist es, wenn eine Pausenerkennungseinrichtung vorgesehen ist, durch die ein Betriebszustand und ein Ruhezustand des Arbeitsgeräts erkennbar sind. Wenn ein Ruhezustand erkannt wurde, der sich über eine vorgegebene Mindestzeitdauer erstreckt, kann die aktuelle Gesamt-Expositionsinformation gelöscht oder zurückgesetzt werden. Die Pausenerkennungseinrichtung ermöglicht es somit, unterschiedliche Arbeitstage zu unterscheiden. Sofern der Bediener eine längere Pause (z. B. wenigstens 8 Stunden) einhält, wird die Gesamt-Expositionsinformation zu Null gesetzt, so dass die Expositionsmessung wieder von neuem beginnt. Wenn hingegen die Pausenzeit zu kurz ist, wird die Expositionsmessung mit dem bereits vorher gespeicherten Wert fortgeführt. Selbstverständlich sind auch Zwischenlösungen denkbar, z. B. dass bei Einhaltung einer Pause von einigen Stunden der Gesamt-Expositionswert zumindest um einen Teil vermindert wird.

Weiterhin vorteilhaft ist eine Schnittstelleneinrichtung zum Übermitteln der aktuellen Gesamt-Expositionsinformation und/oder von Expositionsinformationen aus der Speichereinrichtung an eine externe Datenverarbeitungsvorrichtung. Im Hinblick auf mögliche zunehmende Verpflichtungen für den Arbeitgeber, die Einhaltung der Expositionsgrenzwerte durch seine Arbeitnehmer zu überprüfen, kann es erforderlich werden, dass der Arbeitnehmer täglich die Bediener-Expositionen an den von ihm betriebenen Arbeitsgeräten erfassen muss. Mit Hilfe der Schnittstelleneinrichtung kann er sehr einfach z. B. einen Laptop per Kabel oder auch drahtlos mit dem Arbeitsgerät verbinden und die erforderlichen Expositionsdaten herunterladen. In dem als externe Datenverarbeitungsvorrichtung dienenden Laptop kann mit Hilfe einer entsprechenden Software über längere Zeit ein Protokoll erfasst werden, das dem Arbeitgeber als Beleg für die Einhaltung seiner Pflichten dient.

Selbstverständlich können auch in der Speichereinrichtung des Arbeitsgeräts entsprechende Vorkehrungen getroffen werden, dass die Expositionsdaten auch über längere Zeiträume (Wochen, Monate) gespeichert werden, bis sie gelöscht oder über die Schnittstelleneinrichtung nach außen abgegeben werden.

Bei einer besonders vorteilhaften Ausführungsform der Erfindung ist eine Druckerfassungseinrichtung zum Erfassen eines von dem Bediener an der Körperkontaktstelle ausgeübten Drucks vorgesehen. Es ist bekannt, dass Schädigungen an Hand oder Arm des Bedieners unter anderem auch von der Greifkraft abhängig sind, mit der der Bediener z. B. einen Handgriff des Arbeitsgeräts hält. Je fester der Bediener den Handgriff hält, desto stärker werden die Vibrationen auf die Knochen der Hand übertragen, weil die dazwischen liegenden Weichgewebe der Hand ihre schwingungsentkoppelnde Wirkung nicht mehr entfalten können.

Dementsprechend ist es besonders vorteilhaft, wenn durch die Auswerteeinrichtung die Expositionsinformation in Abhängigkeit von dem durch die Druckerfassungseinrichtung erfassten Druck korrigierbar ist. Hält nämlich der Bediener in einer geeigneten Arbeitssituation, in der das Arbeitsgerät sehr stark schwingt, den Handgriff nur locker mit der Hand, ist seine Exposition verhältnismäßig gering. Dieser Betriebszustand kann von der Auswerteeinrichtung dahingehend genutzt werden, dass nur reduzierte Expositionswerte protokolliert werden, die unter den an sich bei derartigen Beschleunigungen errechenbaren Expositionswerten liegen.

Weiterhin kann die Auswerteeinrichtung derart ausgestaltet werden, dass sie bei Unterschreiten eines vorgegebenen (Minimal-)Grenzwertes durch den von der Druckerfassungseinrichtung erfassten Druck keine Erhöhung der Gesamt-Expositionsinformation bewirkt. Dies ist vor allem für den Fall interessant, dass das Arbeitsgerät eine gewisse Zeit auch ohne Führung durch den Bediener - z. B. im Leerlauf - betrieben werden kann. Dann entstehen am Arbeitsgerät und an der Körperkontaktstelle zwar Vibrationsbeschleunigungen, die jedoch nicht oder nur geringfügig auf den Bediener einwirken. Soweit somit kein oder nur ein geringer Körperkontakt des Bedieners mit dem Arbeitsgerät herrscht, können diese Schwingungen vernachlässigt werden.

Bei einer anderen vorteilhaften Ausführungsform der Erfindung ist eine Kontoeinrichtung zum Führen von getrennten Konten für die Gesamt-Expositionsinformationen von wenigstens zwei Bedienern vorgesehen. Viele Arbeitsgeräte - gerade auf Baustellen - werden von mehreren Bedienern eingesetzt. Eine einfache Ermittlung der Gesamt-Expositionsinformation würde möglicherweise relativ rasch zu dem Hinweis führen, dass der zulässige Grenzwert überschritten wurde, obwohl die zugrunde liegenden Expositionsdaten auf mehrere Bediener verteilt werden müssten und die einzelnen Bediener noch lange nicht den Grenzwert erreicht haben. Mit dem Führen von getrennten Konten ist es möglich, dass jedem Bediener individuell die Exposition zugeschrieben wird, die tatsächlich nur ihn, aber nicht einen anderen Bediener betrifft.

Bei einer besonders vorteilhaften Weiterentwicklung der Erfindung ist eine mit der Kontoeinrichtung zusammenwirkende Bedieneridentifikationseinrichtung vorgesehen, zum Identifizieren des jeweils aktuellen Bedieners und Aktivieren des dem Bediener zugeordneten Kontos derart, dass die jeweiligen Expositionsinformationen der über dieses Konto geführten Gesamt-Expositionsinformation zugeschlagen werden. Die Bedieneridentifikationseinrichtung stellt somit auch bei mehreren Bedienern sicher, dass die jeweilige Exposition nur dem Bediener zugerechnet wird, der auch tatsächlich gerade mit dem Arbeitsgerät arbeitet.

Vorteilhafterweise weist die Bedieneridentifikationseinrichtung eine Umschalteinrichtung auf, mit der eine Bedienerkennung zwischen wenigstens zwei Bedienern umgeschaltet und ein einem Bediener zugeordnetes Bedienerkennungssignal erzeugt werden kann. Anhand des Bedienerkennungssignals kann die Kontoeinrichtung das zugehörige Konto aktivieren.

Alternativ oder ergänzend dazu kann die Bedieneridentifikationseinrichtung eine Empfangseinrichtung aufweisen, mit der ein dem Bediener zugeordnetes Bedienerkennungssignal empfangen werden kann, welches z. B. von einer durch den Bediener getragenen Sendeeinrichtung abgegeben wird. Dazu kann der Bediener die Sendeeinrichtung betätigen, um die Bedieneridentifikationseinrichtung darüber zu informieren, dass nunmehr er das Arbeitsgerät bedienen wird.

Besonders vorteilhaft ist es aber, wenn die Sendeeinrichtung einen Sender mit niedriger Sendeleistung aufweist, insbesondere einen Bluetooth-Sender oder einen Sender mit Transponder, wie z. B. eine RFID-Karte (Smart-Card, RFID-Tag). Aufgrund der niedrigen Sendeleistung ist das Bedienerkennungssignal nur dann empfangbar, wenn sich der Bediener in unmittelbarer Umgebung des Arbeitsgeräts aufhält. Insbesondere bei einer RFID-Karte sendet die am Arbeitsgerät vorhandene Empfangseinrichtung Signale aus, um in ihrer Umgebung abzufragen, ob ein Bediener "vorhanden" ist. Sobald sich ein Bediener mit der z. B. in einer Tasche seiner Bekleidung untergebrachten RFID-Karte dem Arbeitsgerät nähert, wird er erkannt und automatisch das ihm zugeordnete Konto aktiviert. Die verschiedenen Bediener, die nacheinander das gleiche Arbeitsgerät nutzen wollen, müssen dann keine weiteren Maßnahmen ergreifen, um eine korrekte Erfassung einer Exposition zu gewährleisten. Diese einfache, automatisierte Bedienung ist insbesondere auf Baustellen sehr vorteilhaft.

Bei einer besonders vorteilhaften Ausführungsform der Erfindung ist das Arbeitsgerät handgehalten oder -geführt, also z. B. ein Bohr- und/oder Schlaghammer, ein Stampfer, eine Vibrationsplatte, eine Walze oder ein Fugenschneider. An dem Arbeitsgerät sind wenigstens zwei Körperkontaktstellen in Form von Führungsgriffen für jeweils eine Hand des Bedieners vorgesehen, so dass der Bediener das Arbeitsgerät gut halten kann. An jeder der Körperkontaktstellen ist wenigstens ein Beschleunigungsaufnehmer angeordnet. Die Expositionsinformationen sind durch die Auswerteeinrichtung für jede der Körperkontaktstellen separat bestimmbar und zu einer jeweiligen Gesamt-Expositionsinformation integrierbar. Auf diese Weise können für beide Hände des Bedieners getrennt Expositionen bestimmt werden, um eine Überbelastung von nur einer Hand rechtzeitig und zuverlässig erkennen zu können.

Eine weitere vorteilhafte Entwicklung der Erfindung ist gekennzeichnet durch eine Dämpfungsvorgabeeinrichtung zum Eingeben einer Dämpfungsbetriebs-Information durch den Bediener, wenn er zusätzliche Maßnahmen zu seinem Schutz vor Vibrationen außerhalb des Arbeitsgeräts getroffen hat. Dies ist insbesondere dann der Fall, wenn er beim Führen des Arbeitsgeräts Handschuhe trägt, weil ihn die Handschuhe vor der Exposition durch Schwingungen schützen. Bei Vorliegen der Dämpfungsbetriebs-Information sind die Expositionsinformationen durch die Auswerteeinrichtung in einer vorgegebenen Weise derart verminderbar, dass die Gesamt-Expositionsinformation in geringerem Maße ansteigt als in einem Betriebszustand, in dem die Dämpfungsbetriebs-Information nicht vorliegt. Auf diese Weise soll der Schutzmaßnahme des Bedieners Rechnung getragen werden. Wenn er nämlich z. B. durch das Tragen von Handschuhen dafür gesorgt hat, dass geringere Schwingungen in seinen Körper einwirken, werden dementsprechend auch niedrigere Expositionswerte durch die Auswerteeinrichtung protokolliert. Der Grad, um den die Expositionswerte verminderbar sind, kann durch entsprechende Versuche ermittelt werden. In der Praxis ist es z. B. denkbar, dass die Dämpfungsvorgabeeinrichtung eine Taste aufweist, die der Bediener dann betätigt, wenn er Handschuhe trägt, um damit der Expositionsmessvorrichtung die entsprechende Dämpfungsbetriebs-Information zukommen zu lassen.

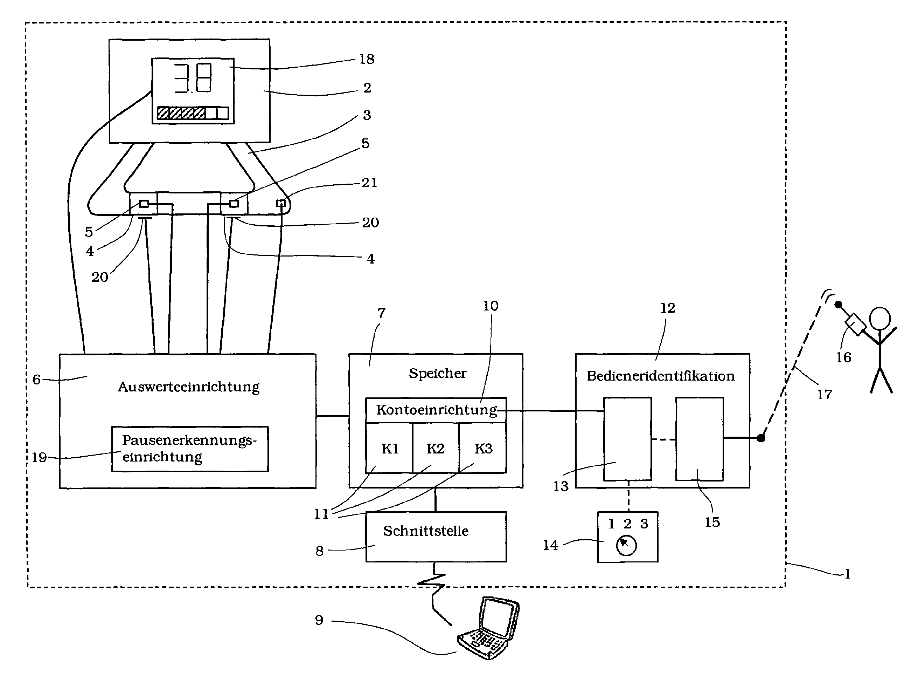

Diese und weitere Merkmale der Erfindung werden nachfolgend anhand eines Beispiels unter Zuhilfenahme der begleitenden Figur erläutert. Die **einzige Figur zeigt** in schematischer Darstellung ein erfindungsgemäßes Arbeitsgerät 1 mit einer Expositionsmessvorrichtung.

Das Arbeitsgerät 1 ist nur in Form eines Kastens dargestellt, um jegliche Art eines von Hand gehaltenen oder führbaren Arbeitsgeräts, aber auch aufsitzbare Arbeitsgeräte zu symbolisieren. Derartige Arbeitsgeräte weisen üblicherweise schwingungserzeugende Einrichtungen auf, wodurch Schwingungen hoher Intensität auf Hände, Arme und Körper der Bedienpersonen übertragen werden. Dies betrifft insbesondere pneumatisch, elektrisch, hydraulisch oder mit Verbrennungsmotor betriebene Geräte, wie Kettensägen, Maschinen mit Schlagwerk (Bohr- und/oder Schlaghammer) oder Schleifer. Schwingungen können darüber hinaus auch durch die Fahrbewegung eines Arbeitsgeräts auf unebenem Boden, aber auch bei Luftfahrzeugen auftreten. Verstärkt wird das Problem bei Arbeitsgeräten, die gezielt zum Erzeugen von Vibrationen eingesetzt werden, wie z. B. Stampfer, Vibrationsplatten und Vibrationswalzen zur Bodenverdichtung. Somit eignet sich die Erfindung für alle Arten von Arbeitsgeräten, bei denen der Bediener derart mit der Maschine in Kontakt steht, dass Vibrationen in seinen Körper eingeleitet werden.

Das beispielhaft gezeigte Arbeitsgerät 1 weist ein Hauptgehäuse 2 und einen daran angebrachten Führungsgriff 3 auf. Auch diese Aufteilung ist nur beispielhaft gewählt, um das Prinzip der Erfindung zu erläutern. Wie sich aus Obigem ergibt, sind selbstverständlich viele weitere Varianten, z. B. mit mehreren Hauptgehäusen 2 oder mehreren Führungsgriffen 3 denkbar. Bei einem Bohrhammer ist es z. B. üblich, wenigstens zwei getrennte Halte- bzw. Führungsgriffe vorzusehen, so dass der Bediener die für ihn jeweils günstigste Griffposition auswählen kann. Der in der Figur gezeigte Führungsgriff 3 kann z. B. ein Führungsgriff an einem Stampfer sein.

Der Führungsgriff 3 weist zwei als Körperkontaktstellen dienende Griffstellen 4 auf, an denen der Bediener den Führungsgriff 3 greifen soll. Die Griffstellen 4 können z. B. durch entsprechend geformte Gummimanschetten gekennzeichnet werden, um den Bediener einen ergonomischen Zugriff zu ermöglichen. Bei Bohrhämmern sind üblicherweise die Führungsgriffe 3 derart ausgestaltet, dass sie vollständig als Griffstelle 4 dienen, so dass der Bediener kaum eine andere Möglichkeit hat, das Gerät an anderer Stelle zu greifen.

An jeder der Griffstellen 4 ist außen oder im Inneren wenigstens ein Beschleunigungsaufnehmer 5 angebracht. Als Beschleunigungsaufnehmer 5 eignen sich verschiedene, an sich bekannte Beschleunigungssensoren, die derart ausgelegt sind, dass sie wenigstens die in der Praxis auftretenden und für die Gesundheitsgefährdung des Bedieners relevanten Schwingungen hinsichtlich ihrer Intensität und Dauer erfassen können. Die Beschleunigungsaufnehmer 5 können Beschleunigungen eindimensional aber auch mehrdimensional, insbesondere räumlich erfassen. Gegebenenfalls sind zu diesem Zweck mehrere Beschleunigungsaufnehmer 5 vorzusehen. Bei der Auswahl und Auslegung der Beschleunigungsaufnehmer 5 kommt es darauf an, möglichst alle relevanten Schwingungen, die in den Körper des Bedieners einwirken können, zu erfassen.

Bei Arbeitsgeräten, bei denen vorwiegend eindimensionale Schwingungen und Stöße auftreten (z. B. bei Meißelhämmern) mag es unter Umständen genügen, nur einen Beschleunigungsaufnehmer 5 zur Erfassung dieser Beschleunigungsrichtung vorzusehen.

Die von dem oder den Beschleunigungsaufnehmern 5 gemessenen Beschleunigungswerte werden an eine Auswerteeinrichtung 6 übertragen. Die Auswerteeinrichtung 6 dient zum fortlaufenden Bestimmen einer Expositionsinformation aufgrund der von dem Beschleunigungsaufnehmer 5 oder weiterer Beschleunigungsaufnehmern erfassten Beschleunigungswerte über eine Zeitdauer der Beschleunigung und zum Summieren bzw. Integrieren der Expositionsinformation zu einer aktuellen Gesamt-Expositionsinformation. In den Normen EN ISO 5349-1 und -2 werden Verfahren zum Messen und Bewerten der Einwirkung von Schwingungen auf das Hand-Arm-System des Menschen beschrieben. Insofern ist die Aufgabe der Auswerteeinrichtung an sich bekannt, so dass sich eine eingehende Beschreibung erübrigt. Zusammenfassend kann die Funktion der Auswerteeinrichtung jedoch wie folgt dargestellt werden:

Die von dem oder den Beschleunigungsaufnehmern 5 gelieferten Beschleunigungs-Messwerte werden quadriert und über der Zeit aufintegriert oder aufsummiert. Die Quadrierung ist sinnvoll, damit höhere Beschleunigungswerte überproportional in das Gesamtergebnis eingehen, da stärkere Beschleunigungen auch überproportionale Schädigungen im menschlichen Körper hervorrufen können. Dadurch lässt sich die Expositionsleistung (Exposition) am Handgriff ermitteln und z. B. durch die Formel

$$\text{Exposition} = \text{Summe} \left( \text{Intensität [in m/s}^2]^2 \times \Delta t \right)$$

darstellen.

Die Aufintegration lässt sich durch dem Fachmann bekannte analoge oder digitale Verfahren erreichen. Beim analogen Integrationsverfahren werden Augenblickswerte über Frequenzfilter gefiltert und einer Echtzeitanalyse unterzogen. Bei einem digitalen Integrationsverfahren werden die gemessenen Beschleunigungswerte über Frequenzfilter ausgewertet und zu Mittelwerten gemittelt, die dann z. B. mit Hilfe einer Fast-Fourier-Transformation (FFT) im passenden Zeittakt aufsummiert werden. Derartige Intergrationsverfahren sind dem Fachmann bekannt, so dass sich eine nähere Darstellung erübrigt.

Mit Hilfe der Auswerteeinrichtung 6 lässt sich somit die tatsächlich im Lauf der Zeit an einem Handgriff (Körperkontaktstelle 4) entstehende Expositionsleistung ermitteln, von der angenommen wird, dass sie in den menschlichen Körper einwirkt.

Es ist bekannt, dass die auf den Körper einwirkenden Frequenzen unterschiedlich starke Schädigungswirkungen haben können. Dazu ist z. B. in der Norm DIN EN ISO 5349-1, Anhang A, die Bereitstellung von Frequenzbewertungs-und Bandbegrenzungsfiltern beschrieben. Derartige Filter sind auch in der Auswerteeinrichtung 6 vorzusehen. Die Frequenzbewertungsfilter bilden die angenommene Bedeutung der einzelnen Frequenzen bei der Verursachung von Schäden der Hand ab, so dass nur die Frequenzen, die eine hohe Schädigung bewirken, in die Aufintegration vollständig Eingang finden, während Frequenzen mit geringerer Schädigungswirkung von Vornherein um einen Faktor vermindert werden. Darüber hinaus ist es möglich, unterschiedliche Frequenzbewertungen für unterschiedliche Schwingungsrichtungen durchzuführen.

Bandbegrenzungsfilter, insbesondere Hochpass- oder Tiefpassfilter blenden den Einfluss von Frequenzen außerhalb eines für die Schädigung interessierenden Frequenzbereichs (z. B. 5,6 Hz bis 1400 Hz) auf den Expositions-Messwert aus.

Die von der Auswerteeinrichtung 6 ermittelten Expositionsinformationen, insbesondere die aktuelle Gesamt-Expositionsinformation, die die Expositionsleistung an der jeweils zugeordneten Griffstelle 4 wiederspiegelt, werden in einer Speichereinrichtung 7 gespeichert. Selbstverständlich kann die Speichereinrichtung 7 auch Zwischenwerte, Werte von anderen Griffstellen 4, Einzelwerte von Beschleunigungsaufnehmern 5 oder Angaben über den Verlauf einer Messung speichern. Dies kann vor allem dann von Bedeutung sein, wenn sehr stark unterschiedliche Schwingungen im Betrieb des Arbeitsgeräts 1 auftreten, insbesondere Schwingungen, die oberhalb eines zulässigen Beschleunigungswertes liegen. Bei Auswertung der Expositionsdaten kann der Arbeitgeber, aber auch der Hersteller des Arbeitsgeräts Rückschlüsse auf Extremsituationen beim Einsatz des Arbeitsgeräts ziehen und entsprechenden Gegenmaßnahmen zum Schutz des Bedieners ergreifen.

Die Speichereinrichtung 7 ist mit einer Schnittstelleneinrichtung 8 gekoppelt, über die die Daten nach außen, z. B. an eine externe Datenverarbeitungsvorrichtung, übermittelt werden können. Bei der Datenverarbeitungsvorrichtung kann es sich z. B. um einen Laptop 9 handeln, der über ein Kabel oder auch über eine Funkstrecke mit der Schnittstelleneinrichtung 8 verbunden wird, um in bestimmten Abständen (täglich, wöchentlich) die Informationen aus der Speichereinrichtung 7 auszulesen. In dem Laptop 9 sollten dann geeignete Programme installiert sein, um Protokolle und Auswertungen erstellen zu können, die den arbeitsrechtlichen Vorschriften entsprechen.

Mit der Speichereinrichtung verbunden bzw. in die Speichereinrichtung 7 integriert kann eine Kontoeinrichtung 10 sein, die mehrere getrennte Konten 11 verwaltet, die jeweils einem Bediener des Arbeitsgeräts zugeordnet sind. In der Figur sind drei Konten K1, K2, K3 (Bezugszeichen 11) gezeigt, die somit drei Bediener repräsentieren. Auf diese Weise ist es möglich, dass für jeden Bediener getrennt eine Gesamt-Exposition ermittelt wird. Selbstverständlich können in den Konten 11 auch Unterkonten für eine tageweise Protokollierung bzw. für die linke und die rechte Hand getrennt vorgesehen werden.

Besonders vorteilhaft wird die Kontoeinrichtung 10 durch eine Bedieneridentifikationseinrichtung 12 angesteuert. Die Bedieneridentifikationseinrichtung 12 dient dazu festzustellen, welcher Bediener gerade das Arbeitsgerät 1 bedient, so dass das ihm zugeordnete Konto 11 aktiviert werden kann und eine korrekte Protokollierung der Exposition hergestellt ist.

Dazu weist die Bedieneridentifikationseinrichtung 12 eine Umschalteinrichtung 13 auf, mit der eine Bedienerkennung zwischen wenigstens zwei Bedienern (im vorliegenden Beispiel drei Bediener) umgeschaltet und ein dem Bediener zugeordnetes Bedienerkennungssignal erzeugt werden kann.

In einfacher Weise ist die Umschalteinrichtung 13 mit einem Drehschalter 14 gekoppelt, der drei Stellungen (für drei Bediener) einnehmen kann. Vor Beginn der Arbeit mit dem Arbeitsgerät muss der Bediener dann den Drehschalter 14 auf die ihm zugeordnete Nummer einstellen, um die korrekte Protokollierung sicherzustellen.

Der Drehschalter 14 kann als Schlüsselschalter, ähnlich wie bei einer Registrierkasse im Gaststättengewerbe, ausgebildet sein, so dass eine Umschaltung der Konten nur mit Hilfe eines Schlüssels durch eine berechtigte Person erfolgen kann.

Alternativ oder ergänzend dazu ist eine automatische Bedienerkennung und Umschaltung der Konten 11 besonders vorteilhaft. Dazu ist in der Bedieneridentifikationseinrichtung 12 eine Empfangseinrichtung 15 vorgesehen, die ein von einer Sendeeinrichtung 16 abgegebenes Bedienerkennungssignal 17 empfängt. Die Sendeeinrichtung 16 wird vom Bediener getragen. Sie kann z. B. eine Taste aufweisen, mit der der Bediener dem Arbeitsgerät 1 das ihm persönlich zugeordnete Bedienerkennungssignal 17 übermitteln kann.

Besonders vorteilhaft ist es aber, wenn die Sendeeinrichtung 16 einen Sender mit niedriger Sendeleistung aufweist, dessen Bedienerkennungssignal 17 nur dann empfangbar ist, wenn sich der Bediener in unmittelbarer Umgebung des Arbeitsgeräts aufhält. Es sind heute Kurzstrecken-Funkübertragungen bekannt, bei denen ein derartiger Sender keine eigene Energieversorgung mehr benötigt. Zum Beispiel kann der Sender in der Sendeeinrichtung 16 aus einem Transpcnder bestehen, der von einem Funksignal von der Empfangseinrichtung 15 erregt wird und ein entsprechendes Antwortsignal als Bedienerkennungssignal 17 an den Empfänger 16 zurückliefert, wenn der Bediener direkt an dem Arbeitsgerät 1 steht. Die Bedieneridentifikationseinrichtung 12 kann dann mit Hilfe der Empfangseinrichtung 15 feststellen, welcher Bediener direkt am Arbeitsgerät 1 steht und dementsprechend tatsächlich auch mit dem Arbeitsgerät 1 arbeitet.

Die Serideeinrichtung 16 kann z. B. als Bluetooth-Sender oder als so genannte RFID-Karte ausgebildet sein, die von dem Bediener lediglich in der Hosentasche getragen werden muss. Immer dann, wenn er sich dem Arbeitsgerät 1 deutlich nähert, erkennt ihn das Arbeitsgerät 1, und aktiviert mit Hilfe der Bedieneridentifikationseinrichtung 12 und der Kontoeinrichtung 10 das ihm zugeordnete Konto 11. Der Bediener muss dann keine weiteren Vorkehrungen mehr treffen, um sicherzustellen, dass die nachfolgend im Betrieb auftretende Exposition seinem persönlichen Konto 11 zugeschrieben wird.

Eine derartige Identifikationsübertragung kann ähnlich arbeiten, wie z. B. das Keyless-Go® -System der Fa. Mercedes-Benz, mit der der Zugang und das Starten von Pkws kontrolliert werden kann, ohne dass der Fahrer einen Zündschlüssel benötigt.

Wesentliche Baugruppen der Expositionsmessvorrichtung, insbesondere die Auswerteeinrichtung 6, die Speichereinrichtung 7 und die Bedieneridentifikationseinrichtung 12 sowie gegebenenfalls die Schnittstelleneinrichtung 8 und der Drehschalter 14 sind in das Arbeitsgerät voll integriert und somit von dem Bediener in der Regel nicht separat wahrnehmbar.

Die von der Auswerteeinrichtung 6 erfassten und in der Speichereinrichtung 7 gespeicherten Expositionsdaten, insbesondere die dem aktuellen Bediener zugeordnete aktuelle Gesamt-Expositionsinformation lässt sich auf einer Anzeige 18 darstellen, die vorzugsweise im Blickbereich des Bedieners liegt. Die Anzeige kann optisch oder akustisch erfolgen.

Eine akustische Anzeige ist insbesondere bei einfacheren Geräten hilfreich, wenn die gemessene Gesamt-Exposition einen vorgegebenen Grenzwert überschreitet. Mit Hilfe des akustischen Warnsignals wird der Bediener darüber informiert, dass er nicht länger mit dem Arbeitsgerät arbeiten sollte, um gesundheitliche Schäden zu vermeiden bzw. zumindest arbeitsrechtliche Vorschriften einzuhalten.

Eine optische Anzeige kann darüber hinaus zusätzliche Informationen bereitstellen. Dazu ist es möglich, einen der aktuellen Gesamt-Exposition entsprechenden Wert als Zahl auszugeben (siehe Figur). Die Zahl kann vom Bediener abgelesen und manuell protokolliert werden, wenn er die Arbeit beendet hat. Dies würde den oben beschriebenen Aufwand mit einer Schnittstelleneinrichtung 8 und einem daran angekoppelten Computer 9 ersetzen.

Besonders interessant ist ein Vergleich des aktuellen Gesamt-Expositionswerts mit einem Grenzwert für eine zulässige Gesamt-Exposition. Dazu wird z. B. in der EU-Richtlinie 2002/44/EG ein täglicher Expositionsgrenzwert, normiert auf einen Bezugszeitraum von 8 Stunden, auf 5 m/s$^2$ festgesetzt. Daraus lässt sich als Bezugsgröße ein Wert von 5$^2$ x 8 m$^2$/s$^4$ x h je Tag = 200 m$^2$/s$^4$ x h errechnen. Bei einheitlicher Verwendung der Einheiten kann somit der Wert "200" als Grenzwert vorgegeben werden, gegenüber dem die aktuell gemessene Gesamt-Exposition abgeglichen wird.

Bei Unter- oder Überschreiten des Grenzwerts kann dem Bediener ein optisches Signal, z. B. mit Hilfe einer grünen und einer roten Leuchtdiode, übermittelt werden. Ebenso ist es möglich, das Verhältnis der aktuellen Gesamt-Exposition zu dem maximalen Grenzwert darzustellen, z. B. mit Hilfe eines aus Leuchtdioden bestehenden Leuchtbandes (siehe Figur, unterer Teil der Anzeige 18). Wenn alle Leuchtdioden aufleuchten, muss der Bediener die Arbeit einstellen, weil die maximal zulässige Belastung erreicht ist. Umgekehrt können die Leuchtdioden nacheinander mit fortschreitender Arbeit erlöschen, bis keine Leuchtdiode mehr aufleuchtet und der Bediener den Betrieb einstellen muss.

Ergänzend oder zusätzlich dazu kann anhand der vorliegenden Informationen auch eine Restbetriebszeit errechnet werden, die dem Bediener auf der Anzeige 18 mitgeteilt wird. Als Grundlage dafür wird außer dem maximal zulässigen Grenzwert und der bis dahin summierten Gesamt-Exposition ein Mittelwert über die Zeit bestimmt und für die Zukunft hochgerechnet. Das zugrunde liegende Verfahren ähnelt der Berechnung einer Restreichweite bei einem Pkw, bei dem unter Berücksichtigung des noch im Tank befindlichen restlichen Kraftstoffs und des aktuellen Verbrauchs die noch zur Verfügung stehende Reichweite berechnet wird.

Die Auswerteeinrichtung 6 kann weiterhin eine Pauseneinrichtung 19 aufweisen, mit deren Hilfe erkannt wird, wie lange der Bediener seine Arbeit unterbrochen hat. Dies ist insbesondere dafür wichtig, dass zu Beginn eines neuen Arbeitstags die Gesamt-Exposition von neuem, also von Null beginnend, ermittelt werden muss. Die Pausenerkennungseinrichtung 19 kann für diesen Zweck eine Uhr aufweisen, mit der der Beginn eines neuen Arbeitstags erkannt wird. Da jedoch ein Baustellenbetrieb auch bei Nacht möglich sein kann, ist die Synchronisation auf einen Arbeitstag oft nur bedingt möglich. Daher kann als maßgebliches Kriterium für das Rück- bzw. Nullsetzen der Gesamt-Exposition eine längere Arbeitsunterbrechung (z. B. 8 Stunden) gewählt werden. Selbstverständlich muss die Pause auch wieder einem einzelnen Bediener über die Kontoeinrichtung 10 zugeordnet werden, um eine Arbeitsunterbrechung für nur diesen Bediener zu erkennen.

Die Auswerteeinrichtung 6 mit der Pauseneinrichtung 19 und die Speichereinrichtung 7 sollten eine nicht dargestellte Spannungsversorgung aufweisen, die sicherstellt, dass die ermittelten Expositionsdaten zumindest über einige Tage gespeichert werden können.

Es ist bekannt, dass die Schädigung der Hand durch Schwingungen an einem Führungsgriff unter anderem auch abhängig von der Kraft sind, mit der der Bediener den Führungsgriff hält. Bei geringer Greifkraft wirken die an der Hand befindlichen Weichgewebe schwingungsentkoppelnd bzw. dämpfend, so dass die Knochen in Hand und Arm geringer beansprucht werden.

Daher können an den Griffstellen 4 Drucksensoren 20, die als Druckerfassungseinrichtung dienen, angeordnet werden. Die Drucksensoren 20 ermitteln, ob die Hand des Bedieners die Griffstelle 4 mit normaler, überhöhter oder niedriger Greifkraft hält. Dementsprechend können die zu diesem Zeitpunkt gemessenen Beschleunigungswerte in der Auswerteeinrichtung mit einem Korrekturfaktor belegt werden.

Die Messwerte der Drucksensoren 20 werden ebenfalls der Auswerteeinrichtung 6 zugeführt.

Zudem können die Drucksensoren 20 erkennen, ob nur eine Hand oder beide Hände exponiert sind, d. h. ob der Bediener das Arbeitsgerät 1 mit einer oder mit beiden Händen hält. Anhand dieser Information kann die Auswerteeinrichtung 6 die Expositionsdaten für jede Hand getrennt aufintegrieren und gegebenenfalls auch nur für eine Hand des Bedieners anzeigen, dass der zulässige Expositions-Grenzwert überschritten wurde. Dabei ist zu beachten, dass insbesondere bei Hand-Arm-Schwingungen Schädigungen lokal auftreten können und sich nicht auf den gesamten Körper beziehen.

Weiterhin kann es vorteilhaft sein, wenn eine Dämpfungsvorgabeeinrichtung 21 vorgesehen ist, die z. B. in Form eines Tasters oder Schalters an dem Führungsgriff 3 angebracht werden kann. Mit Hilfe der Dämpfungsvorgabeeinrichtung 21 kann der Bediener dem Arbeitsgerät 1, insbesondere der mit der Dämpfungsvorgabeeinrichtung 21 verbundenen Auswerteeinrichtung 6 mitteilen, dass er zusätzliche Maßnahmen zu seinem Schutz vor Vibrationen außerhalb des Arbeitsgeräts 1 getroffen hat. Dazu gehört z. B. das Tragen von Handschuhen, die die Hände des Bedieners gegenüber von außen einwirkenden Schwingungen isolieren. Die dann auf die Hand wirkenden Schwingungen sind geringer, was bei der Ermittlung der Gesamt-Exposition berücksichtigt werden sollte.

Bei Vorliegen einer durch Dämpfungsvorgabeeinrichtung 21 abgegebenen Dämpfungsbetriebs-Information können durch die Auswerteeinrichtung 6 die gemessenen Beschleunigungswerte in einer vorgegebenen Weise vermindert werden. Dementsprechend erhöht sich die Gesamt-Exposition weniger schnell als ohne Dämpfungsbetriebs-Information, also wenn der Bediener z. B. keine Schutzhandschuhe trägt.

Es sollte sichergestellt sein, dass die Dämpfungsvorgabeeinrichtung 21 vor jeder Inbetriebnahme neu betätigt werden muss, damit die Auswerteeinrichtung 6 nicht versehentlich davon ausgeht, dass ein Dämpfungsbetrieb vorliegt, obwohl der Bediener keine Schutzmaßnahmen ergriffen hat.

Die erfindungsgemäße Expositionsmessvorrichtung wurde oben anhand eines Beispiels für ein handgeführtes Arbeitsgerät erläutert. Selbstverständlich eignet sie sich auch für Arbeitsgeräte, die im Aufsitzbetrieb zu führen sind und bei denen Schwingungen in den gesamten Körper des Bedieners eingeleitet werden.

Die erfindungsgemäße Expositionsmessvorrichtung ist fester Bestandteil des Arbeitsgeräts 1. Anders als bei den in den Normen DIN EN ISO 5349-1 und -2 beschriebenen Verfahren und Vorrichtungen muss sie also nicht separat an dem Arbeitsgerät 1 befestigt werden. Vielmehr wird die Expositionsmessvorrichtung schon vom Hersteller des Arbeitsgeräts 1 eingebaut. Die erforderlichen Sensoren sind derart in das Arbeitsgerät 1 integriert, dass sie auch im Betrieb keinen Schaden nehmen bzw. von dem Bediener nicht separat wahrgenommen werden können.

Bei der Gesamtkörperexposition ist zu berücksichtigen, dass zukünftig mit unterschiedlichen Grenzwerten und Filtern für Vertikal- und Horizontalbeschleunigungen zu rechnen ist. Diesem kann dadurch Rechnung getragen werden, dass die in der entsprechenden Richtung gemessenen Beschleunigungswerte z. B. am Sitz getrennt frequenzbewertet und dann vektoriell addiert und anschließend integriert werden, entsprechend den dann gültigen Vorschriften.

**Patentansprüche**

1.  Expositionsmessvorrichtung für ein Arbeitsgerät (1), mit

    - wenigstens einem Beschleunigungsaufnehmer (5), der an einer Körperkontaktstelle (4) angeordnet ist, an der ein Bediener das Arbeitsgerät (1) durch Halten, Führen oder Aufsitzen berührt, wobei der Beschleunigungsaufnehmer (5) zum Erfassen eines aktuellen Beschleunigungswertes an der Körperkontaktstelle (4) dient;
    - einer Auswerteeinrichtung (6) zum fortlaufenden Bestimmen einer Expositionsinformation aufgrund der von dem Beschleunigungsaufnehmer (5) erfassten Beschleunigungswerte über einer Zeitdauer der Beschleunigung und zum Summieren oder Integrieren der Expositionsinformationen zu einer aktuellen Gesamt-Expositionsinformation; und mit
    - einer Speichereinrichtung (7) zum Speichern der aktuellen Gesamt-Expositionsinformation und/oder einzelner Expositionsinformationen;

    wobei die Expositionsmessvorrichtung in das Arbeitsgerät (1) integriert ist.

2.  Expositionsmessvorrichtung nach Anspruch 1, **dadurch gekennzeichnet, dass**

    - an der Körperkontaktstelle (4) mehrere Beschleunigungsaufnehmer (5) angeordnet sind; und dass
    - die von den Beschleunigungsaufnehmern (5) erfassten Beschleunigungswerte durch die Auswerteeinrichtung (6) zu einer Expositionsinformation zusammenfassbar sind.

3.  Expositionsmessvorrichtung nach Anspruch 1 oder 2, **gekennzeichnet durch** eine Anzeigeeinrichtung (18) zum Anzeigen eines Zustands der aktuellen Gesamt-Expositionsinformation.

4.  Expositionsmessvorrichtung nach Anspruch 3, **dadurch gekennzeichnet, dass** der Zustand der aktuellen Gesamt-Expositionsinformation ein Zustand ist, ausgewählt aus der Gruppe:

    - Unter- oder Überschreiten eines Grenzwertes für eine zulässige Gesamt-Exposition (maximal zulässiger Gesamt-Expositionswert), insbesondere eines täglichen Expositionsgrenzwertes durch die aktuelle Gesamt-Expositionsinformation;
    - ein der aktuellen Gesamt-Expositionsinformation entsprechender Zahlenwert;
    - ein Verhältnis des Zahlenwerts der aktuellen Gesamt-Expositionsinformation zu dem maximal zulässigen Gesamt-Expositionswert;
    - die verbleibende Differenz zwischen der aktuellen Gesamt-Expositionsinformation und dem maximal zulässigen Gesamt-Expositionswert; und/oder
    - eine der verbleibenden Differenz zwischen der aktuellen Gesamt-Expositionsinformation und dem maximal zulässigen Gesamt-Expositionswert entsprechende Restbetriebszeit des Arbeitsgeräts bis zum Erreichen des maximal zulässigen Gesamt-Expositionswerts.

5.  Expositionsmessvorrichtung nach einem der Ansprüche 2 bis 4, **dadurch gekennzeichnet, dass** die Anzeigeeinrichtung (18) zum Ausgeben eines optischen oder akustischen Signals ausgebildet ist.

6.  Expositionsmessvorrichtung nach einem der Ansprüche 1 bis 5, **gekennzeichnet durch** eine Pausenerkennungseinrichtung (19), **durch** die ein Betriebszustand und ein Ruhezustand des Arbeitsgeräts (1) erkennbar sind und **durch** die die aktuelle Gesamt-Expositionsinformation löschbar oder zurücksetzbar ist, wenn ein Ruhezustand erkannt wurde, der sich über eine vorgegebene Mindestzeitdauer erstreckt.

7. Expositionsmessvorrichtung nach einem der Ansprüche 1 bis 6, **gekennzeichnet durch** eine Schnittstelleneinrichtung (8) zum Übermitteln der aktuellen Gesamt-Expositionsinformation und/oder von Expositionsinformationen aus der Speichereinrichtung (7) an eine externe Datenverarbeitungsvorrichtung (9).

8. Expositionsmessvorrichtung nach einem der Ansprüche 1 bis 7, **gekennzeichnet durch** eine Druckerfassungseinrichtung (20) zum Erfassen eines von dem Bediener an der Körperkontaktstelle (4) ausgeübten Drucks.

9. Expositionsmessvorrichtung nach Anspruch 8, **dadurch gekennzeichnet, dass** durch die Auswerteeinrichtung (6) die Expositionsinformation in Abhängigkeit von dem durch die Druckerfassungseinrichtung (20) erfassten Druck korrigierbar ist.

10. Expositionsmessvorrichtung nach Anspruch 8 oder 9, **dadurch gekennzeichnet, dass** die Auswerteeinrichtung (6) bei Unterschreiten eines vorgegebenen Grenzwertes durch den von der Druckerfassungseinrichtung (20) erfassten Drucks keine Erhöhung der Gesamt-Expositionsinformation bewirkt.

11. Expositionsmessvorrichtung nach einem der Ansprüche 1 bis 10, **gekennzeichnet durch** eine Kontoeinrichtung (10) zum Führen von getrennten Konten (11) für die Gesamt-Expositionsinformationen von wenigstens zwei Bedienern.

12. Expositionsmessvorrichtung nach Anspruch 11, **gekennzeichnet durch** eine mit der Kontoeinrichtung (10) zusammenwirkende Bedieneridentifikationseinrichtung (12) zum Identifizieren des jeweils aktuellen Bedieners und Aktivieren des dem Bediener zugeordneten Kontos (11) derart, dass die jeweiligen Expositionsinformationen der über dieses Konto (11) geführten Gesamt-Expositionsinformation zugeschlagen werden.

13. Expositionsmessvorrichtung nach Anspruch 12, **dadurch gekennzeichnet, dass** die Bedieneridentifikationseinrichtung (12) eine Umschalteinrichtung (13) aufweist, zum Umschalten einer Bedienerkennung zwischen wenigstens zwei Bedienern und Erzeugen eines einem Bediener zugeordneten Bedienerkennungssignals.

14. Expositionsmessvorrichtung nach Anspruch 12 oder 13, **dadurch gekennzeichnet, dass** die Bedieneridentifikationseinrichtung (12) eine Empfangseinrichtung (15) aufweist, zum Empfangen eines einem Bediener zugeordneten Bedienerkennungssignals (17).

15. Expositionsmessvorrichtung nach Anspruch 13 oder 14, **dadurch gekennzeichnet, dass** das Bedienerkennungssignal (17) von einer durch den Bediener tragbaren Sendeeinrichtung (16) abgebbar ist.

16. Expositionsmessvorrichtung nach Anspruch 15, **dadurch gekennzeichnet, dass** die Sendeeinrichtung (16) einen Sender mit niedriger Sendeleistung, insbesondere einen Bluetooth-Sender, einen Transponder oder eine RFID-Karte aufweist, derart, dass das Bedienerkennungssignal (17) nur dann empfangbar ist, wenn sich der Bediener in unmittelbarer Umgebung des Arbeitsgeräts (1) aufhält.

17. Expositionsmessvorrichtung nach einem der Ansprüche 1 bis 16, **dadurch gekennzeichnet, dass**

  - das Arbeitsgerät (1) handgehalten oder -geführt ist;
  - wenigstens zwei Körperkontaktstellen (4) für jeweils eine Hand des Bedieners vorgesehen sind, an denen jeweils wenigstens ein Beschleunigungsaufnehmer (5) angeordnet ist; und dass
  - die Expositionsinformationen durch die Auswerteeinrichtung (6) für jede der Körperkontaktstellen (4) separat bestimmbar und zu einer jeweiligen Gesamt-Expositionsinformation integrierbar sind.

18. Expositionsmessvorrichtung nach einem der Ansprüche 1 bis 17, **gekennzeichnet durch** eine Dämpfungsvorgabeeinrichtung (21) zum Eingeben einer Dämpfungsbetriebs-Information **durch** den Bediener, wenn zusätzliche Maßnahmen zum Schutz des Bedieners vor Vibrationen außerhalb des Arbeitsgeräts (1) getroffen sind, wobei bei Vorliegen der Dämpfungsbetriebs-Information die Expositionsinformationen **durch** die Auswerteeinrichtung (6) in einer vorgegebenen Weise verminderbar sind, derart, dass die Gesamt-Expositionsinformation in geringerem Maße ansteigt als in einem Betriebszustand, in dem die Dämpfungsbetriebs-Information nicht vorliegt.